# EUROPEAN PATENT APPLICATION

(11) **EP 2 789 350 A2**
(43) Date of publication of application: **15.10.2014**
(21) Application number: 14164048.2
(22) Date of filing: 09.04.2014
(51) Int. Cl.: A61L 9/014, B01D 39/20, B01D 46/00, B01D 53/04, F25D 17/04, B01D 53/48, B01D 53/54, B01J 20/28

(54) **Deodorizing filter and refrigerator having the same**

(30) Priority: 09.04.2013 KR 20130038500
(71) Applicant: Samsung Electronics Co., Ltd., Suwon-si, Gyeonggi-do 443-742 (KR)
(72) Inventor: CHOI, Jung Min, Gyeonggi-do (KR); LEE, Jong Ho, Gyeonggi-do (KR); YOO, Chae Hyun, Gyeonggi-do (KR); KIM, Hyun Sook, Gyeonggi-do (KR); LIM, Jung Soo, Gyeonggi-do (KR)
(74) Representative: Walaski, Jan Filip

(57) **Abstract**

A deodorizing filter including at least one deodorizing member to adsorb odor particles contained in fluid. The deodorizing member includes a substrate having plural pass-through pores to allow passage of the fluid, an adherent material applied to a surface of the substrate, and plural porous deodorizer materials fixed to the surface of the substrate by the adherent material to adsorb the odor particles. The deodorizing filter more effectively deodorizes interior air of a refrigerator owing to an increased contact area between interior air of the refrigerator and the deodorizer materials of the deodorizing filter.

## Description

The present invention relates to a deodorizing filter in which granular activated charcoal is fixed to a wire mesh and a refrigerator having the same.

Generally, a refrigerator is an electronic device that includes a storage compartment for storage of food and a cold air supply device for supply of cold air, and keeps food fresh.

Food stored in the refrigerator tends to diffuse unique odors. For example, fermented food, such as Kimchi, cheese, etc., diffuse a unique odor, and fish emits the unique odor of fish. Moreover, food may spoil when stored for a long time, thus diffusing unpleasant odors.

For this reason, the refrigerator generally includes a deodorizing filter to remove unpleasant odor within the refrigerator. Conventional deodorizing filters have been fabricated by mixing a deodorizer material, such as, e.g., activated charcoal, with a bonding agent for the deodorizer material, and drying the mixture.

However, the deodorizing filters in the mixture of the deodorizer material and the bonding agent may have difficulty in achieving sufficient deodorization because of a small contact area between interior air of the refrigerator and the deodorizer material.

It is an aspect of the present disclosure to provide a deodorizing filter in which granular activated charcoal is fixed to a wire mesh and a refrigerator having the same. Additional aspects of the disclosure will be set forth in part in the description which follows and, in part, will be apparent from the description, or may be learned by practice of the disclosure.

In accordance with one aspect of the disclosure, a deodorizing filter includes at least one deodorizing member to adsorb odor particles contained in fluid, wherein the deodorizing member includes a substrate having plural pass-through pores to allow passage of the fluid, an adherent material applied to a surface of the substrate, and plural porous deodorizer materials fixed to the surface of the substrate by the adherent material to adsorb the odor particles.

Spaces to allow passage of the fluid having passed through the pores of the substrate may be defined between the porous deodorizer materials.

The substrate may be a wire mesh.

The adherent material may be applied to wires of the mesh to fix the porous deodorizer materials to the wires of the mesh.

The deodorizing filter may further include a porous packaging member surrounding the at least one deodorizing member.

Each of the deodorizer materials may include at least one of activated charcoal, carbon fiber, zeolite, and silica.

Each of the deodorizer materials may be formed by applying a metal oxide or metal sulfate to at least one of activated charcoal, carbon fiber, zeolite, and silica.

The metal oxide or the metal sulfate may include at least one of manganese oxide (MnO), copper oxide (CuO), and copper sulfate (CuSO₄).

Each of the deodorizer materials may include activated charcoal formed by applying an organic acid or an inorganic acid to at least one of activated charcoal, carbon fiber, zeolite, and silica.

The organic acid or the inorganic acid may include at least one of sulfuric acid (H₂SO₄) and phosphoric acid (H₃PO₄).

In accordance with another aspect of the disclosure, a refrigerator includes at least one storage compartment, a cooling device to cool the storage compartment, and a deodorizing module to adsorb odor particles contained in interior air of the storage compartment, wherein the deodorizing module includes a flow path for the flow of the air, a deodorizing filter including a substrate having plural pass-through pores to allow passage of the air, an adherent material applied to a surface of the substrate, and plural porous deodorizer materials fixed to the surface of the substrate by the adherent material to adsorb the odor particles, and a deodorizing fan installed in the flow path to move the air such that the air passes through the deodorizing filter.

The deodorizing module may further include a filter receptacle in which the deodorizing filter is received, and the deodorizing filter may be attachable to or detachable from the filter receptacle.

The deodorizing fan may be located downstream of the deodorizing filter, and may move the air having passed through the deodorizing filter into the storage compartment. The refrigerator may further include a display unit to display a replacement time point of the deodorizing filter.

The refrigerator may further include a controller to control the display unit so as to display replacement of the deodorizing filter when a drive time of the deodorizing fan is a preset reference time or more.

The refrigerator may further include an odor particle concentration detection unit to detect a concentration of odor particles contained in the interior air of the storage compartment.

The refrigerator may further include a controller to change a drive rate of the deodorizing fan based on a detection result of the odor particle concentration detection unit.

The controller may increase the drive rate of the deodorizing fan when the concentration of odor particles is a first reference concentration or more.

The controller may reduce the drive rate of the deodorizing fan when the concentration of odor particles is a second reference concentration or less.

In accordance with a further aspect of the disclosure, a refrigerator includes a storage compartment, a cooling duct to supply cold air into the storage compartment, an evaporator installed in the cooling duct to cool interior air of the cooling duct via evaporation of refrigerant, a cooling fan to discharge the cooled air into the storage compartment, a suction port, through which interior air of the storage compartment is introduced into the cooling duct, and a deodorizing filter fitted into the suction port to adsorb odor particles contained in the air to be introduced into the cooling duct, wherein the deodorizing filter includes a substrate having plural pass-through pores to allow passage of the air, an adherent material applied to a surface of the substrate, and plural porous deodorizer materials fixed to the surface of the substrate by the adherent material to adsorb the odor particles.

The refrigerator may further include a filter receptacle in which the deodorizing filter is received, and the deodorizing filter may be attachable to or detachable from the filter receptacle.

The refrigerator may further include a display unit to display a replacement time point of the deodorizing filter.

The refrigerator may further include a controller to control the display unit so as to display replacement of the deodorizing filter when a drive time of the deodorizing fan is a preset reference time or more.

These and/or other aspects of the disclosure will become apparent and more readily appreciated from the following description of the embodiments, taken in conjunction with the accompanying drawings of which:
FIG. 1A is a view showing an external appearance of a deodorizing filter in accordance with one embodiment of the disclosure;
FIG. 1B is a view showing an external appearance of a deodorizing member included in the deodorizing filter in accordance with the embodiment of the disclosure;
FIG. 1C is an enlarged view of portion A of FIG. 1B;
FIGS. 2A to 2D are views showing a manufacturing process of the deodorizing filter in accordance with the embodiment of the disclosure;
FIG. 3 is a view showing an external appearance of an exemplary refrigerator having the deodorizing filter in accordance with the embodiment of the disclosure;
FIG. 4 is a front view showing the exemplary refrigerator having the deodorizing filter in accordance with the embodiment of the disclosure;
FIG. 5 is a view showing a cooling device of the exemplary refrigerator having the deodorizing filter in accordance with the embodiment of the disclosure;
FIGS. 6A and 6B are perspective views showing an external appearance of an exemplary deodorizing module including the deodorizing filter in accordance with the embodiment of the disclosure;
FIGS. 7A and 7B are exploded perspective views showing the interior of the exemplary deodorizing module including the deodorizing filter in accordance with the embodiment of the disclosure;
FIG. 8 is a view showing the flow of air in the exemplary deodorizing module including the deodorizing filter in accordance with the embodiment of the disclosure;
FIG. 9 is a block diagram showing control flow of the exemplary refrigerator having the deodorizing filter in accordance with the embodiment of the disclosure;
FIG. 10 is a detailed view showing a control panel shown in FIG. 3;
FIG. 11 is a flowchart showing a method of operating the deodorizing module by the exemplary refrigerator having the deodorizing filter in accordance with the embodiment of the disclosure;
FIG. 12 is a flowchart showing a method of displaying replacement of the deodorizing filter by the exemplary refrigerator having the deodorizing filter in accordance with the embodiment of the disclosure;
FIG. 13 is view showing an external appearance of another exemplary refrigerator having the deodorizing filter in accordance with the embodiment of the disclosure;
FIG. 14 is a front view showing the another exemplary refrigerator having the deodorizing filter in accordance with the embodiment of the disclosure;
FIG. 15 is an enlarged view of portion B of FIG. 14;
FIG. 16 is a view showing a cooling device of the another exemplary refrigerator having the deodorizing filter in accordance with the embodiment of the disclosure;
FIG. 17 is a block diagram showing control flow of the another exemplary refrigerator having the deodorizing filter in accordance with the embodiment of the disclosure;
FIG. 18 is a detailed view showing a control panel shown in FIG. 13; and
FIG. 19 is a flowchart showing a method of displaying replacement of the deodorizing filter by the another exemplary refrigerator having the deodorizing filter in accordance with the embodiment of the disclosure.

Reference will now be made in detail to the embodiments of the present disclosure, examples of which are illustrated in the accompanying drawings, wherein like reference numerals refer to like elements throughout.

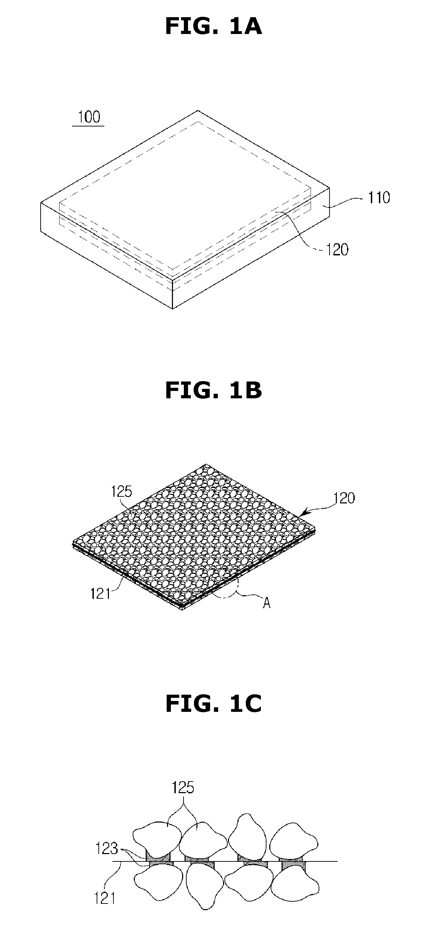

FIG. 1A is a view showing an external appearance of a deodorizing filter in accordance with one embodiment of the disclosure, FIG. 1B is a view showing an external appearance of a deodorizing member included in the deodorizing filter in accordance with the embodiment of the disclosure, and FIG. 1C is an enlarged view of portion A of FIG. 1B.

Referring to FIG. 1A, the deodorizing filter 100 includes a plurality of deodorizing members 120 to adsorb odor particles contained in air, and a packaging member 110 surrounding the deodorizing members 120 to bind the same without a risk of separation.

The packaging member 110 is formed of a non-woven fabric having plural pass-through pores for the flow of air. The non-woven fabric may be felt made by arranging fibers in different directions and bonding the same using a synthetic resin adhesive, and include the pass-through pores to allow fluid to pass through the non-woven fabric. However, a material of the packaging member 110 is not limited to the non-woven fabric, and any other materials having pass-through pores, such as a wire mesh, may be used to form the packaging member 110.

In addition, an antibiotic material, such as silver ions (Ag+) and others, may be applied to the packaging member 110, to assist deodorization by the deodorizing members 120 that will be described hereinafter and sterilization of floating bacteria contained in air.

Referring to FIGS. 1B and 1C, each of the deodorizing members 120 includes plural porous deodorizer materials 125 to adsorb odor particles contained in air, a substrate 121 to which the porous deodorizer materials 125 are fixed, the substrate 121 having plural pass-through pores for passage of the air containing odor particles, and an adherent material 123 to adhere the porous deodorizer materials 125 and the substrate 121 to each other.

The porous deodorizer materials 125 are acquired by applying an adsorbent material, which is specialized to adsorb specific odor particles, to a porous base material. In this case, the specialized adsorbent material, applied to the porous base material, has a weight percent of 0.1% to 10% based on the total weight of the porous deodorizer materials 125.

The porous base material may be a material having innumerable fine pores, such as granular activated charcoal, carbon fibers, carbon sheets, granular silica, zeolite, and others.

In particular, activated charcoal is an amorphous aggregate of carbon particles having innumerable fine pores, and has an important feature that well defined fine pores provide a great inner surface area of 1000 m2 per 1g of activated charcoal. Thus, odor particles may be easily adsorbed onto the surface of the activated charcoal owing to the innumerable fine pores and the great inner surface area.

The specialized adsorbent material may be a metal oxide or a metal sulfate to diffuse odor particles including sulfur that causes odor of spoiled food, or an organic acid or inorganic acid for reaction with odor particles including amines that cause the unique odor of fish.

Representative odor that may be generated from food is odor generated as food spoils or is fermented, or odor of fish. A material including sulfur, more particularly, methyl mercaptan (CH₄S) is known as a representative material causing odor due to spoilage or fermentation. Methyl mercaptan has a boiling point of 5.95°C, and may cause unpleasant odor by being vaporized under refrigeration. In addition, a material including amines, more particularly, trimethyl amine (CH₃)₃N is known as a representative material causing odor of fish.

Copper oxide (CuO) as one kind of a metal oxide decomposes methyl mercaptan (CH₄S), known as a representative unpleasant odor causing material, into dimethyl disulfide (CH₃SSCH₃). That is, the copper oxide does not participate in dehydrogenation reaction in which a pair of methyl mercaptans is condensed into dimethyl disulfide, but serves as a catalyst to induce condensation from methyl mercaptans into dimethyl disulfide. Here, although dimethyl disulfide as well as methyl mercaptan are known as unpleasant odor causing materials, dimethyl disulfide has a boiling point of 190°C and is present in liquid phase at room temperature or under refrigeration, thus not causing unpleasant odor under refrigeration.

Other materials for decomposition of methyl mercaptan may include manganese oxide (MnO) and copper sulfate (CuSO₄).

Sulfuric acid (H₂SO₄) as one kind of an inorganic acid generates a quaternary ammonium salt ((CH₃)₄NSO₄) via reaction with trimethyl amine ((CH₃)₃N) known as an unpleasant odor causing material. Trimethyl amine is a basic material and generates a salt (quaternary ammonium salt) via acid-base reaction with an acid material such as sulfuric acid.

Materials to generate the quaternary ammonium salts via reaction with trimethyl amine may include inorganic acids, such as sulfuric acid, phosphoric acid (H₃PO₄), etc., organic acids, and others that may perform acid-base reaction with trimethyl amine.

The substrate 121 serves to fix the above described porous deodorizer materials 125 thereto. The substrate 121 may have any form including plural pass-through pores for the flow of air. In one embodiment of the disclosure, the substrate 121 takes the form of a urethane wire mesh.

The adherent material 123 serves to adhere the porous deodorizer materials 125 and the substrate 121 to each other. The adherent material 123 is applied to the substrate 121 to adhere the porous deodorizer materials 125 to the substrate 121. In the case in which the substrate 121 takes the form of a wire mesh, the adherent material 123 is applied only to wires of the mesh, and thus does not prevent the flow of air. That is, spaces for the passage of air are defined between the porous deodorizer materials 125, providing an increased contact surface area between air passing through the deodorizing member 120 and the porous deodorizer materials 125. This may ensure that odor particles contained in the air are easily adsorbed onto the porous deodorizer materials 125.

More specifically, while the porous deodorizer materials 125 are densely arranged at top and bottom surfaces of the substrate 121 as exemplarily shown in FIG. 1B, spaces for the passage of air are defined between the porous deodorizer materials 125 as exemplarily shown in FIG. 1C. In the case in which air flows from a bottom surface of the deodorizing member 120 to a top surface of the deodorizing member 120, the air may pass through the spaces defined between the porous deodorizer materials 125 fixed to the bottom surface of the substrate 121 while sufficiently coming into contact with surfaces of the porous deodorizer materials 125. Thereafter, the air, having passed through the pass-through pores formed in the substrate 121, may again pass through the spaces defined between the porous deodorizer materials 125 fixed to the top surface of the substrate 121 while sufficiently coming into contact with surfaces of the porous deodorizer materials 125.

In the case of the deodorizing filter 100 described above, when the deodorizing filter 100 reaches a replacement point in time thereof, a user may simply reproduce the deodorizing filter 100 by washing the deodorizing filter 100.

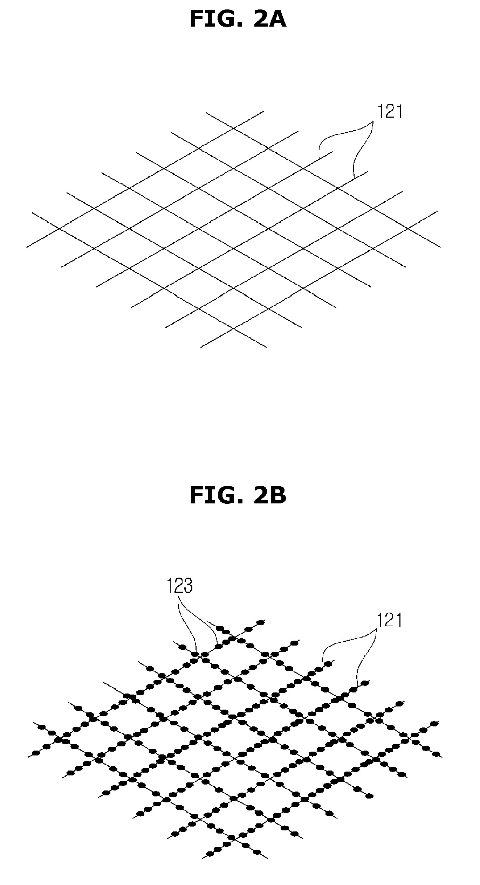

FIGS. 2A to 2D are views showing a manufacturing process of the deodorizing filter in accordance with the embodiment of the disclosure.

First, the substrate 121 having pass-through pores is fabricated. As exemplarily shown in FIG. 2A, a wire mesh may be used as the substrate 121.

Next, the adherent material 123 is applied to both surfaces of the substrate 121. Application of the adherent material 123 to the surfaces of the substrate 121 may be implemented by ejecting the adherent material 123 to the substrate 121, or by dipping the substrate 121 in a container receiving the adherent material 123.

In the case in which the substrate 121 takes the form of a wire mesh, as exemplarily shown in FIG. 2B, the adherent material 123 is applied to wires of the mesh so as not to block the pass-through pores between the wires.

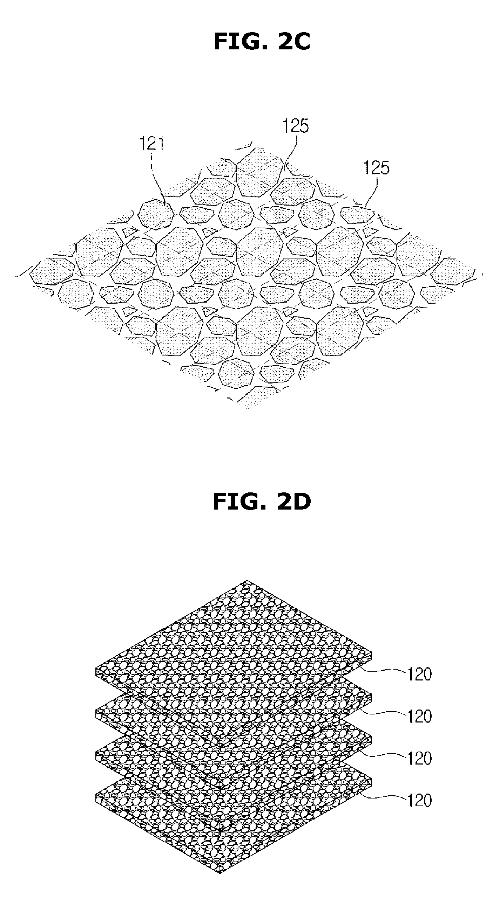

Next, the porous deodorizer materials 125 are attached to the surfaces of the substrate 121 and are dried to be adhered to the substrate 121. In the case in which the substrate 121 takes the form of a wire mesh, as exemplarily shown in FIG. 2C, the substrate 121 and the adherent material 123 do not prevent the flow of air through the deodorizing member 120, and a contact area between the air passing through the deodorizing member 120 and the porous deodorizer materials 125 is maximized. Next, the deodorizing member 120 is cut to an appropriate size.

Next, as a plurality of deodorizing members 120, each of which is manufactured by the above described method, is stacked one above another as exemplarily shown in FIG. 2D, and thereafter is surrounded by the packaging member 110, manufacture the deodorizing filter 100 is completed.

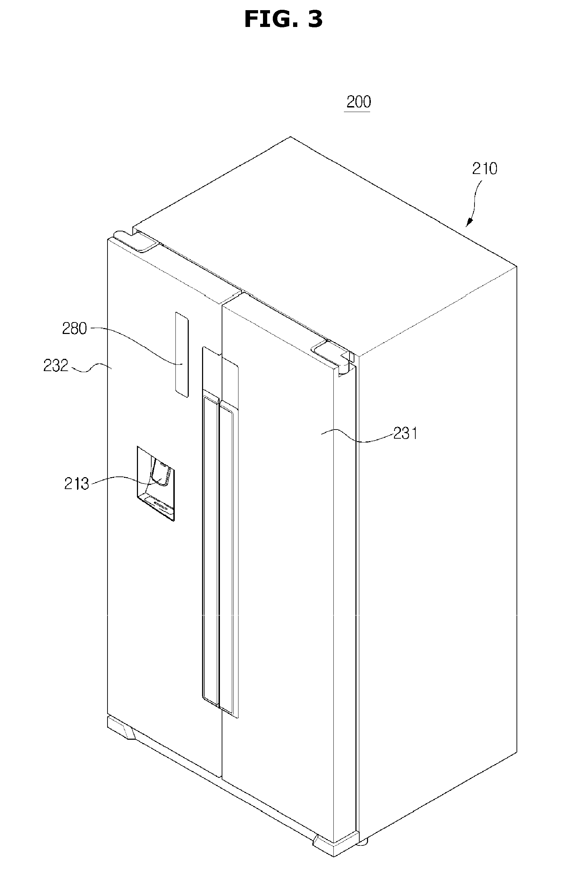

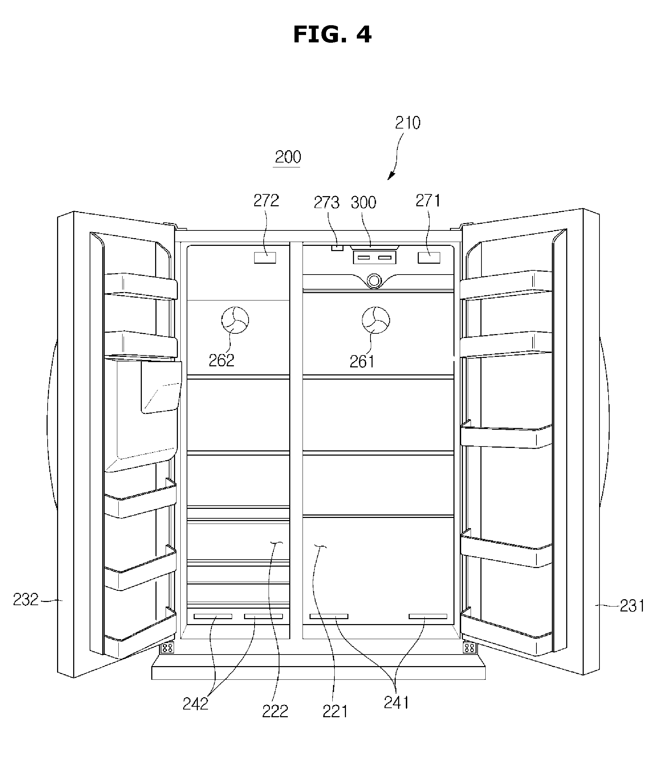

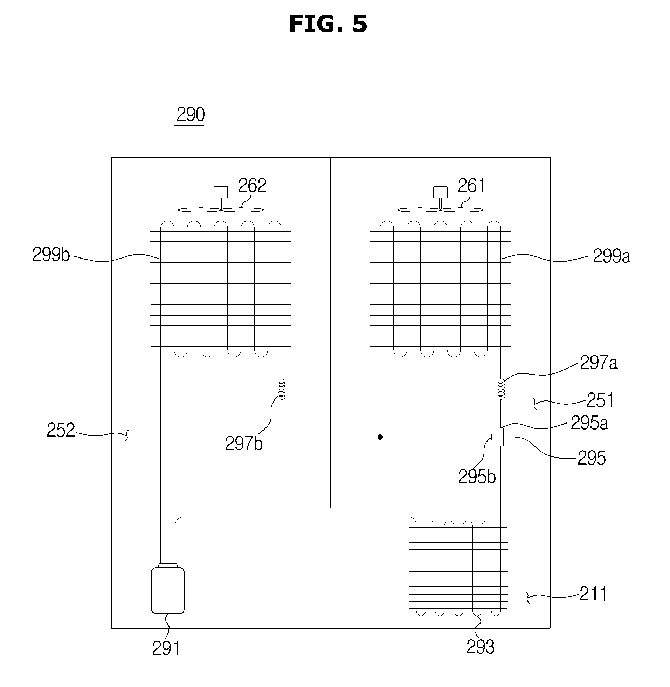

FIG. 3 is a view showing an external appearance of an exemplary refrigerator having the deodorizing filter in accordance with the embodiment of the disclosure, FIG. 4 is a front view showing the exemplary refrigerator having the deodorizing filter in accordance with the embodiment of the disclosure, and FIG. 5 is a view showing a cooling device of the exemplary refrigerator having the deodorizing filter in accordance with the embodiment of the disclosure.

Referring to FIGS. 3 to 5, the refrigerator 200 includes a main body 210 defining an external appearance of the refrigerator 200, storage compartments 221, 222 defined in the main body 210 for storage of food, doors 231, 232 to shield the storage compartments 221, 222 from the outside, and a cooling device 290 to cool the storage compartments 221, 222.

The main body 210 includes an inner shell defining the storage compartments 221, 222, an outer shell coupled to the exterior of the inner shell to define the external appearance of the refrigerator 200, and an insulator interposed between the inner shell and the outer shell to insulate the storage compartments 221, 222 from the outside. A machine room 211 is defined below the main body 210 and some components of the cooling device 290 are installed in the machine room 211.

The storage compartments 221, 222 are separate left and right compartments with an intermediate partition therebetween. The storage compartments 221, 222 include a refrigerating compartment 221 in which food is kept at a temperature slightly above freezing and a freezing compartment 222 in which food is kept below freezing. Both the refrigerating compartment 221 and the freezing compartment 222 have open front faces to allow the user to introduce food into or retrieve food from the storage compartments 221, 222.

The storage compartments 221, 222 are respectively provided with temperature sensors 271, 272 to detect temperatures of the storage compartments 221, 222. The temperature sensors 271, 272 include the first temperature sensor 271 installed in the refrigerating compartment 221 to detect a temperature of the refrigerating compartment 221, and the second temperature sensor 272 installed in the freezing compartment 222 to detect a temperature of the freezing compartment 222. These temperature sensors 271, 272 may be thermistors, electrical resistance of which varies based on temperature.

A gas sensor 273 is installed in an upper region of the refrigerating compartment 221 to detect the concentration of odor particles floating in the refrigerating compartment 221. The gas sensor 273 may be a semiconductor type gas sensor in which two electrodes encapsulated by metal oxide pellets are heated. In the semiconductor type gas sensor, when gas comes into contact with a semiconductor device, gas particles are adsorbed onto the surface of the semiconductor device, which causes movement of free electrons in the semiconductor device, resulting in increased electrical conductivity of the semiconductor device. The concentration of gas may be detected from increase in the electrical conductivity of the semiconductor device. Naturally, the gas sensor 273 is not limited to the semiconductor type gas sensor, and may be a contact combustion type gas sensor, a membrane electrode type gas sensor, a constant-potential electrolytic gas sensor, or the like.

Cooling ducts 251, 252 are installed to rear surfaces of the storage compartments 221, 222 such that interior air of the storage compartments 221, 222 flow through the cooling ducts 251, 252, and are respectively provided with evaporators 299a, 299b that will be described hereinafter. The storage compartments 221, 222 have suction ports 241, 242 formed near the bottom thereof, from which air of the storage compartments 221, 222 is suctioned into the cooling ducts 251, 252. Cooling fans 261, 262 to cause air of the cooling ducts 251, 252 to be discharged into the storage compartments 221, 222 are installed in upper regions of the storage compartments 221, 222. More specifically, air of the refrigerating compartment 221 is suctioned into the first cooling duct 251 through the first suction port 241, and is cooled by the first evaporator 299a that will be described hereinafter while flowing through the first cooling duct 251, and thereafter is discharged into the refrigerating compartment 221 by the first cooling fan 261. In addition, air of the freezing compartment 222 is suctioned into the second cooling duct 252 through the second suction port 242, and is cooled by the second evaporator 299b that will be described hereinafter while flowing through the second cooling duct 252, and thereafter is discharged into the freezing compartment 222 by the second cooling fan 262.

The doors 231, 232 are provided at the front face of the main body 210 to shield the storage compartments 221, 222 from the outside. In addition, the doors 231, 232 are provided at front faces thereof with a dispenser 213 to discharge purified water and a control panel 280 to receive an operating instruction for the refrigerator 200 from the user and display operating information of the refrigerator 200. The control panel 280 will be described below in detail.

The cooling device 290 includes a compressor 291, a condenser 293, a flow path switching valve 295, expansion valves 297a, 297b, and the evaporators 299a, 299b. The compressor 291 is installed in the machine room 211 and serves to compress gas-phase refrigerant, evaporated by the evaporators 299a, 299b, to a high pressure using torque of a motor (not shown) that is rotated upon receiving electrical energy from an external power source, and to pump the compressed refrigerant into the condenser 293. The motor (not shown) of the compressor 291 rotates a rotating shaft via magnetic interaction between a rotor and a stator upon receiving drive current from a drive unit that will be described hereinafter. The motor may be an induction AC servomotor, a synchronized AC servomotor, a Brushless Direct Current (BLDC) motor, or the like. The refrigerant may circulate through the condenser 293, the expansion valves 297a, 297b, and the evaporators 299a, 299b to implement heat-exchange by pressure generated by the compressor 291.

The condenser 293 condenses the high-pressure gas-phase refrigerant compressed by the compressor 291. In other words, the high-pressure gas-phase refrigerant is condensed into liquid-phase refrigerant while passing through the condenser 293. During this condensation, the refrigerant emits latent heat. Latent heat of refrigerant refers to thermal energy emitted to outside air as gas-phase refrigerant, cooled to boiling point thereof, is changed into liquid-phase refrigerant of the same temperature. In addition, thermal energy absorbed from outside air as liquid-phase refrigerant, heated to boiling point thereof, is changed into gas-phase refrigerant of the same temperature is also referred to as latent heat.

The flow path switching valve 295 controls the flow of refrigerant. More specifically, the flow path switching valve 295 may open a first refrigerant exit 295a to allow the refrigerant to pass through both the first evaporator 299a that cools the refrigerating compartment 221 and the second evaporator 299b that cools the freezing compartment 222, and may open a second refrigerant exit 295b to allow the refrigerant to pass through only the second evaporator 299b. In other words, when attempting to cool the refrigerating compartment 221, the refrigerator 200 may open the first refrigerant exit 295a of the flow path switching valve 295 to allow the refrigerant to pass through both the first evaporator 299a and the second evaporator 299b. When attempting to cool the freezing compartment 222, the refrigerator 200 may open the second refrigerant exit 295b of the flow path switching valve 295 to allow the refrigerant to pass through only the second evaporator 299b.

The expansion valves 297a, 297b are arranged downstream of the flow path switching valve 295, and depressurize the liquid-phase refrigerant condensed by the condenser 293. The expansion valves 297a, 297b include the first expansion valve 297a located downstream of the first refrigerant exit 295a to depressurize the refrigerant to be introduced into the first evaporator 299a, and the second expansion valve 297b located downstream of the second refrigerant exit 295b to depressurize the refrigerant to be introduced into the second evaporator 299b.

The expansion valves 297a, 297b depressurize the liquid-phase refrigerant to an evaporable pressure via throttling. Throttling refers to pressure reduction of fluid passing through a narrow flow path, such as a nozzle or an orifice.

The evaporators 299a, 299b are installed in the above described cooling ducts 251, 252, and include the first evaporator 299a installed in the first cooling duct 251 to cool air of the refrigerating compartment 221 and the second evaporator 299b installed in the second cooling duct 252 to cool air of the freezing compartment 222. The evaporators 299a, 299b evaporate the liquid-phase refrigerant depressurized by the expansion valves 297a, 297b, thereby cooling air of the cooling ducts 251, 252. In other words, the refrigerant absorbs latent heat from the outside while being evaporated in the evaporators 299a, 299b, thereby cooling air around the evaporators 299a, 299b.

As the gas-phase refrigerant evaporated by the evaporators 299a, 299b is again introduced into the compressor 291 so as to be compressed by the compressor 291, the refrigerant is circulated through the cooling device 290.

A deodorizing module 300 is installed in an upper region of the refrigerating compartment 221 to adsorb odor particles contained in interior air of the refrigerating compartment 221.

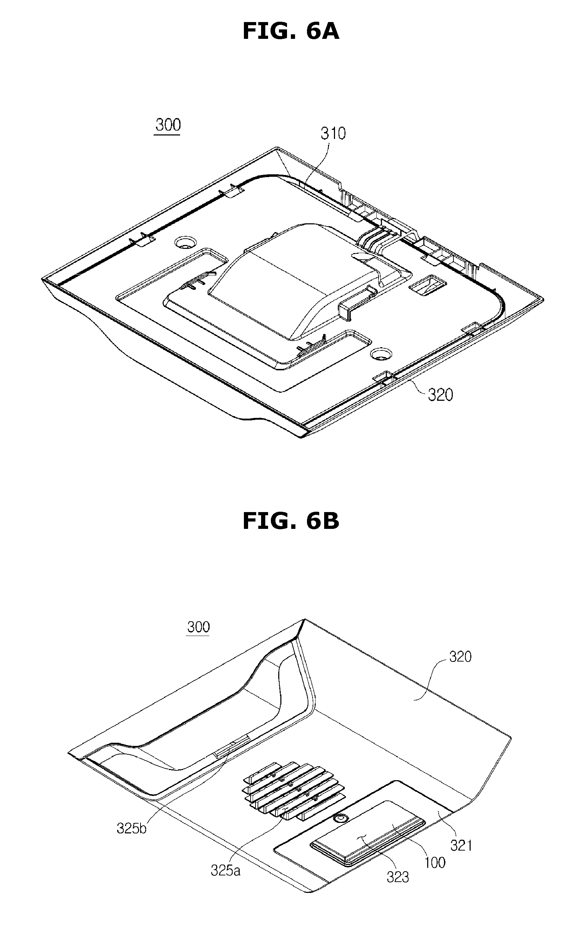

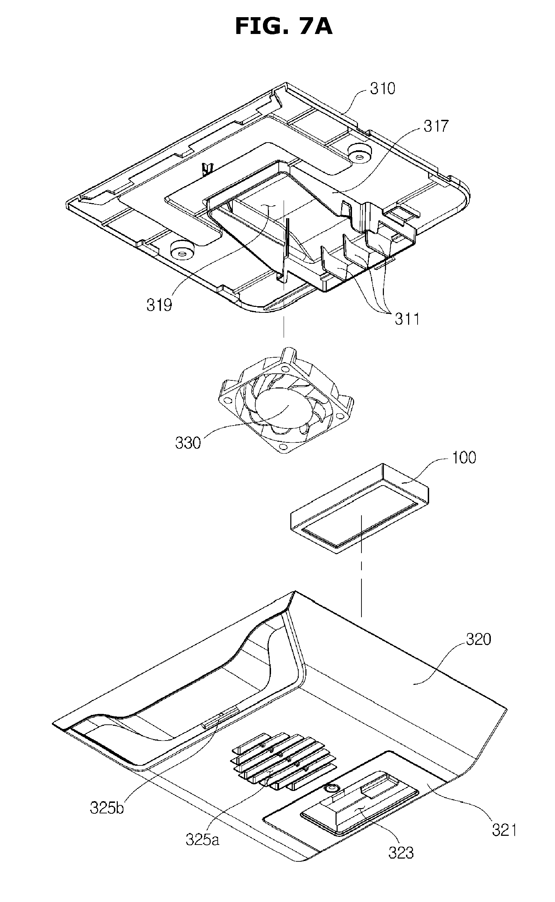

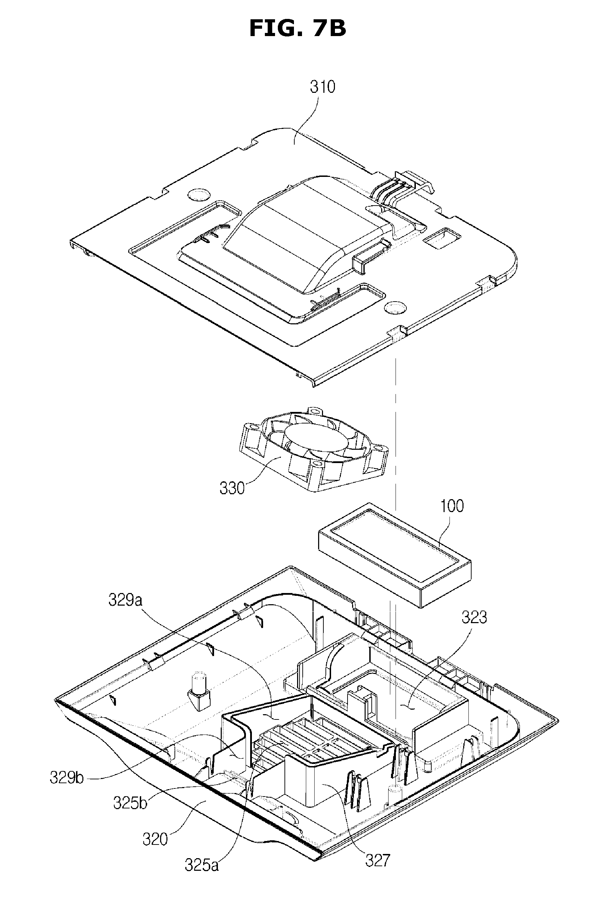

FIGS. 6A and 6B are perspective views showing an external appearance of an exemplary deodorizing module including the deodorizing filter in accordance with the embodiment of the disclosure, and FIGS. 7A and 7B are exploded perspective views showing the interior of the exemplary deodorizing module including the deodorizing filter in accordance with the embodiment of the disclosure.

Referring to FIGS. 6A and 6B and FIGS. 7A and 7B, the deodorizing module 300 includes the deodorizing filter 100 to adsorb odor particles contained in interior air of the refrigerating compartment 221, a deodorizing fan 330 to move the interior air of the refrigerating compartment 221 into the deodorizing module 300, a deodorizing module housing 320 in which the deodorizing filter 100 and the deodorizing fan 300 are received, and a deodorizing module cover 310 to cover the top of the deodorizing module housing 320.

The deodorizing module housing 320 includes a deodorizing filter separation/coupling cover 321 to enable introduction of the deodorizing filter 100 into the deodorizing module housing 320 or withdrawal of the deodorizing filter 100 from the deodorizing module housing 320, an inlet 323 formed in the deodorizing filter separation/coupling cover 321 for introduction of the interior air of the refrigerating compartment 221 into the deodorizing module 300, a first outlet 325a formed in a front region of a bottom face of the deodorizing module 300 for discharge of deodorized air into the refrigerating compartment 221, and a second outlet 325b formed in a lower region of a front face of the deodorizing module 300 for discharge of deodorized air into the refrigerating compartment 221.

The deodorizing filter separation/coupling cover 321 is located at a rear region of the bottom face of the deodorizing module housing 320, and has a size equal to or slightly greater than that of the deodorizing filter 100. In addition, the deodorizing filter separation/coupling cover 321 has one side fixed to the deodorizing module housing 320, and is pivotally rotatable about the fixed side as a rotating axis.

The user may open or close the deodorizing filter separation/coupling cover 321 by pivotally rotating the deodorizing filter separation/coupling cover 321 fixed to the deodorizing module housing 320. In other words, the user may open the deodorizing filter separation/coupling cover 321 to enable introduction or withdrawal of the deodorizing filter 100 without disassembling the deodorizing module 300, and may close the deodorizing filter separation/coupling cover 321 to ensure that the deodorizing filter 100 is stably mounted in the deodorizing module 300.

The inlet 323 is formed in the deodorizing filter separation/coupling cover 321. In addition, the deodorizing module housing 320 is internally provided with a first deodorizing fan fixing member 327 to fix the deodorizing fan 330 from the bottom thereof, a first air passage 329a to guide air forcibly moved by the deodorizing fan 330 to the first outlet 325a, and a second air passage 329b to guide air forcibly moved by the deodorizing fan 330 to the second outlet 325b.

The first deodorizing fan fixing member 327 allows the deodorizing fan 330 to be tilted rearward of the deodorizing module 300. That is, the first deodorizing fan fixing member 327 takes the form of a remaining lower portion of a rectangular box that is obliquely cut downward and rearward. The first deodorizing fan fixing member 327 has an opening formed in a front face thereof.

The first air passage 329a is surrounded by the first deodorizing fan fixing member 327, and guides air forcibly moved by the deodorizing fan 330 to the first outlet 325a formed in the bottom face of the deodorizing module 300.

The second air passage 329b is defined by the opening formed in the front face of the first deodorizing fan fixing member 327, and guides air forcibly moved by the deodorizing fan 330 to the second outlet 325b formed in the front face of the deodorizing module 300.

The deodorizing module cover 310 includes deodorizing filter fixing members 311 to fix the deodorizing filter 100, a second deodorizing fan fixing member 317 to fix the deodorizing fan 330 from the top, and a third air passage 319 to guide air deodorized by the deodorizing filter 100 to the deodorizing fan 330.

The deodorizing filter fixing members 311 protrude downward from the deodorizing module cover 310 to fix the deodorizing filter 100. That is, in a closed state of the deodorizing filter separation/coupling cover 321 described above, the deodorizing filter 100 is fixed by the deodorizing filter fixing members 311 above the deodorizing filter 100 and the deodorizing filter separation/coupling cover 321 below the deodorizing filter 100.

The second deodorizing fan fixing member 317 allows the deodorizing fan 330 to be tilted rearward of the deodorizing module 300 in cooperation with the above described first deodorizing fan fixing member 327. That is, the second deodorizing fan fixing member 317 takes the form of a remaining upper portion of a rectangular box that is obliquely cut rearward and downward, and is engaged with the first deodorizing fan fixing member 327 to fix the deodorizing fan 330 without a risk of movement of the deodorizing fan 330. In addition, the second deodorizing fan fixing member 317 has an opening formed in a rear face thereof.

The third air passage 319 is surrounded by the second deodorizing fan fixing member 317 to guide air deodorized by the deodorizing filter 100 to the deodorizing fan 330. In particular, the third air passage 319 has rounded front and rear regions, and a center region of the third air passage 319 is upwardly concave. Through this shape of the third air passage 319, air introduced through the inlet 323 formed in the bottom face of the deodorizing module 300 smoothly moves along the third air passage 319, thereby being discharged through the first outlet 325a formed in the bottom face of the deodorizing module 300.

The deodorizing filter 100 is located above the inlet 323 such that air introduced through the inlet 323 passes through the deodorizing filter 100. The deodorizing filter 100 adsorbs odor particles contained in the air introduced through the inlet 323. A configuration and a manufacturing method of the deodorizing filter 100 have been described above with reference to FIGS. 1A to 1C and 2A to 2D, and thus a description thereof will be omitted hereinafter.

The deodorizing fan 330 is located between the first air passage 329a and the third air passage 319 and is tilted rearward of the deodorizing module 300 by the first deodorizing fan fixing member 327 and the second deodorizing fan fixing member 317. In addition, the deodorizing fan 330 forcibly moves air of the third air passage 319 into the first and second air passages 329a, 329b, thereby allowing interior air of the refrigerating compartment 221 to be introduced through the inlet 323 and also allowing air deodorized by the deodorizing filter 100 to be discharged through the first and second outlets 325a, 325b.

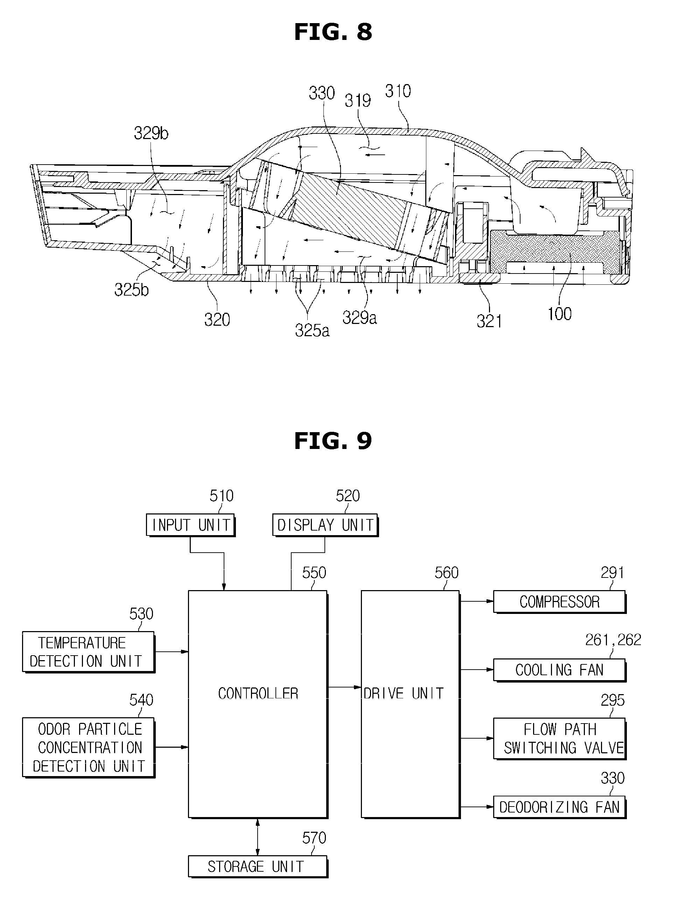

FIG. 8 is a view showing the flow of air in the exemplary deodorizing module including the deodorizing filter in accordance with the embodiment of the disclosure. Referring to FIG. 8, first, interior air of the refrigerating compartment 221 is introduced into the deodorizing module 300 through the inlet 323.

The air introduced through the inlet 323 is deodorized while passing through the deodorizing filter 100. That is, various odor particles contained in the air are adsorbed onto the deodorizing filter 100.

The air having passed through the deodorizing filter 100 is moved to the deodorizing fan 330 along the third air passage 319. As described above, the concave region of the third air passage 319 allows the air having passed through the deodorizing filter 100 to smoothly move to the deodorizing fan 330.

The air moved along the third air passage 319 is moved along the first air passage 329a by the deodorizing fan 330, thereby being discharged into the refrigerating compartment 221 through the first outlet 325a, or is moved along the second air passage 329b, thereby being discharged into the refrigerating compartment 221 through the second outlet 325b.

In summary, the air deodorized by the deodorizing filter 100 is discharged to the outside of the deodorizing module 300 by the deodorizing fan 330.

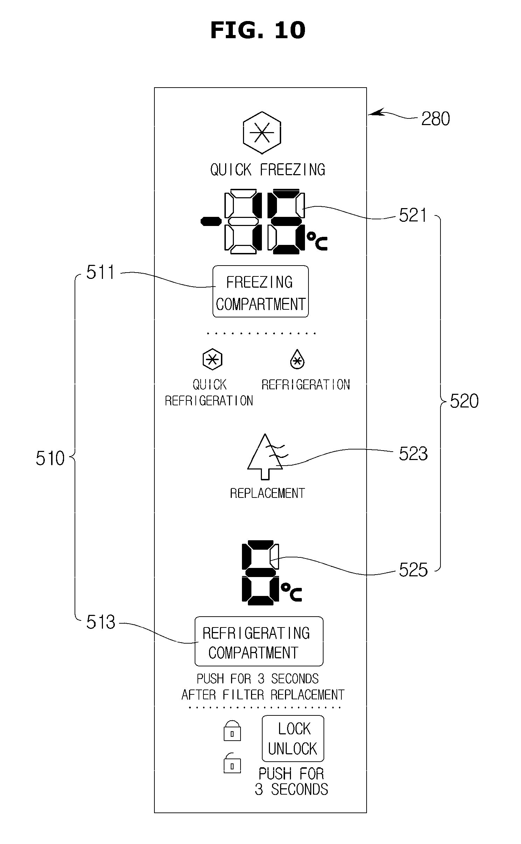

FIG. 9 is a block diagram showing control flow of the exemplary refrigerator having the deodorizing filter in accordance with the embodiment of the disclosure, and FIG. 10 is a detailed view showing the control panel shown in FIG. 3.

Referring to FIGS. 9 and 10, to perform a function thereof, the refrigerator 200 includes an input unit 510, a display unit 520, a temperature detection unit 530, an odor particle concentration detection unit 540, a controller 550, a drive unit 560, a storage unit 570, the compressor 291, the cooling fans 261, 262, the flow path switching valve 295, and the deodorizing fan 330.

A description of the compressor 291, the cooling fans 261, 262, the flow path switching valve 295, and the deodorizing fan 330 will be omitted because these components have been described above.

The display unit 520 and the input unit 510 that will be described hereinafter constitute the control panel 280, and the display unit 520 displays operating information of the refrigerator 200.

The display unit 520 includes a freezing-compartment temperature display area 521 to display a temperature of the freezing compartment 222, a refrigerating-compartment temperature display area 525 to display a temperature of the refrigerating compartment 221, and a deodorizing-filter replacement display area 523 to display a replacement point in time of the deodorizing filter 100. The respective display areas included in the display unit 520 may be a Liquid Crystal Display (LCD) device, a Light Emitting Diode (LED) display device, or the like.

Along with the display unit 520 described above, the input unit 510 constitutes the control panel 280, and receives an operating instruction for the refrigerator 200 from the user.

The input unit 510 includes a freezing-compartment operating-instruction input button 511 to set a target temperature of the freezing compartment 222 and an operating mode of the freezing compartment 222, and a refrigerating-compartment operating-instruction input button 513 to set a target temperature of the refrigerating compartment 221 and an operating mode of the refrigerating compartment 221. The respective input buttons included in the input unit 510 may button type switches, membrane switches, touch pads, or the like.

When the deodorizing-filter replacement display area 523 displays an instruction to replace the deodorizing filter 100, the user may initialize a deodorizing-filter replacement point in time via the refrigerating-compartment operating-instruction input button 513. For example, when the deodorizing-filter replacement display area 523 of the display unit 520 displays "Replacement" of the deodorizing filter 100, the user may initialize a replacement point in time of the deodorizing filter 100 by pushing the refrigerating-compartment operating-instruction input button 513 for several seconds after replacing the deodorizing filter 100 of the deodorizing module 300.

The temperature detection unit 530 includes the first temperature sensor 271 and the second temperature sensor 272 installed respectively in the refrigerating compartment 221 and the freezing compartment 222. The temperature detection unit 530 detects temperatures of the storage compartments 221, 222 and outputs signals corresponding to the detected temperatures to the controller 550.

The odor particle concentration detection unit 540 includes the gas sensor 273 installed in the refrigerating compartment 221, and serves to detect the concentration of odor particles contained in interior air of the refrigerating compartment 221 and to output a signal corresponding to the detected concentration to the controller 550.

The drive unit 560 generates drive current to drive the compressor 291, the cooling fans 261, 262, and the deodorizing fan 330 and drive current to open or close the flow path switching valve 295 based on a control signal of the controller 550 that will be described hereinafter. The drive unit 560 may be an inverter that generates drive current, a pulse width of which is modulated based on an input signal.

In particular, the drive unit 560 may drive the deodorizing fain 330 at various rates by modulating the pulse width of drive current to be applied to the deodorizing fan 330. More specifically, in the case of a first drive rate, the drive unit 560 applies drive current to the deodorizing fan 330 for a time of 1/3 of one period. In the case of a second drive rate, the drive unit 560 applies drive current to the deodorizing fan 330 for a time of 2/3 of one period. In addition, in the case of a third drive rate, the drive unit 560 continuously applies drive current to the deodorizing fan 330. In summary, drive current having a duty rate of 33% is applied to the deodorizing fan 330 at the first drive rate, drive current having a duty rate of 67% is applied to the deodorizing fan 330 at the second drive rate, and drive current having a duty rate of 100% is applied to the deodorizing fan 330 at the third drive rate.

The storage unit 570 may store programs and data to control the refrigerator 200 and may also temporarily store operating information of the refrigerator 200. The storage unit 570 may be a non-volatile memory, such as a magnetic disc, a semiconductor solid state drive, and others, or may be a volatile memory, such as a D-RAM, S-RAM, and others.

The controller 550 controls overall operation of the refrigerator 200. More specifically, the controller 550 generates a control signal to drive the compressor 291 and to open or close the flow path switching valve 295 based on a detection result of the temperature detection unit 530, and transmits the same to the drive unit 560. For example, when the detection result of the temperature detection unit 530 shows that a temperature of the refrigerating compartment 221 is less than a target temperature, the controller 550 transmits a control signal, required to open the first refrigerant exit (295a, FIG. 5) of the flow path switching valve 295 to and drive the compressor 291, to the drive unit 560. When the detection result of the temperature detection unit 530 shows that a temperature of the freezing compartment 222 is less than a target temperature, the controller 550 transmits a control signal, required to open the second refrigerant exit (295b, FIG. 5) of the flow path switching valve 295 and to drive the compressor 291, to the drive unit 560.

The controller 550 generates a control signal to determine a drive rate of the deodorizing fan 330 based on a detection result of the odor particle concentration detection unit 540 and to drive the deodorizing fan 330 based on the determined drive rate, and transmits the same to the drive unit 560. For example, when the detection result of the odor particle concentration detection unit 540 shows that a concentration of odor particles is less than a first reference concentration, the controller 550 transmits a control signal, required to drive the deodorizing fan 330 at the first drive rate, to the drive unit 560. When the detection result of the odor particle concentration detection unit 540 shows that a concentration of odor particles is the first reference concentration or more and is less than a second reference concentration, the controller 550 transmits a control signal, required to drive the deodorizing fan 330 at the second drive rate, to the drive unit 560. In addition, when the detection result of the odor particle concentration detection unit 540 shows that a concentration of odor particles is the second reference concentration or more, the controller 550 transmits a control signal, required to drive the deodorizing fan 330 at the third drive rate, to the drive unit 560.

In addition, the controller 550 compares a cumulative drive time of the deodorizing fan 330 with a reference time, and controls the display unit 520 to display "Replacement" of the deodorizing filter 100 when the cumulative drive time of the deodorizing fan 330 is the reference time or more. Here, the cumulative drive time refers to a total driven time of the deodorizing fan 330 after the deodorizing filter 100 is replaced, and may be calculated from a total time during which the drive unit 560 applies drive current to the deodorizing fan 330.

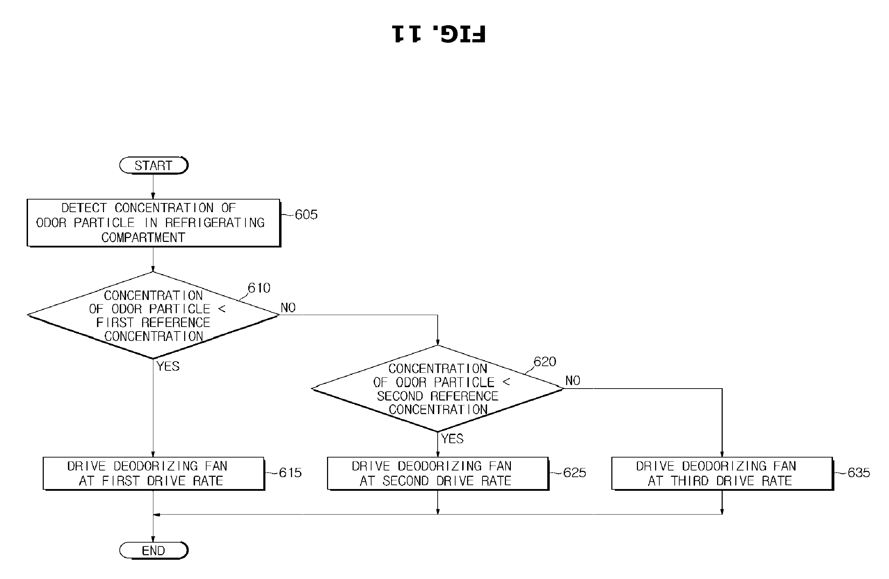

FIG. 11 is a flowchart showing a method of driving the deodorizing module by the refrigerator in accordance with the embodiment of the disclosure.

Referring to FIG. 11, first, the refrigerator 200 detects a concentration of odor particles in the refrigerating compartment 221 via the odor particle concentration detection unit 540 (605).

Next, the refrigerator 200 compares the detected concentration of odor particles with the first reference concentration (610).

When the detected concentration of odor particles is less than the first reference concentration, the refrigerator 200 drives the deodorizing fan 330 at the first drive rate (615).

When the detected concentration of odor particles is the first reference concentration or more, the refrigerator 200 compares the detected concentration of odor particles with the second reference concentration (620).

When the detected concentration of odor particles is less than the second reference concentration, the refrigerator 200 drives the deodorizing fan 330 at the second drive rate (625).

When the detected concentration of odor particles is the second reference concentration or more, the refrigerator 200 drives the deodorizing fan 330 at the third drive rate (635).

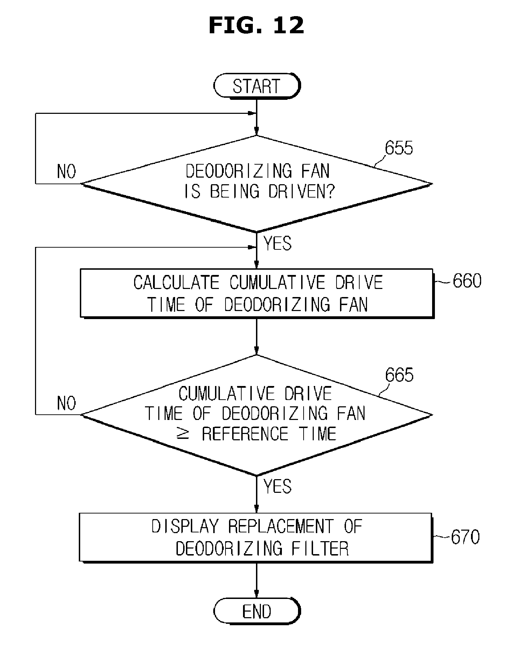

FIG. 12 is a flowchart showing a method of displaying replacement of the deodorizing filter by the exemplary refrigerator having the deodorizing filter in accordance with the embodiment of the disclosure.

Referring to FIG. 12, the refrigerator 200 judges whether or not the deodorizing fan 300 is being driven (655).

When the deodorizing fan 330 is driven, the refrigerator 200 calculates a cumulative drive time of the deodorizing fan 330 (660).

Next, the refrigerator 200 compares the cumulative drive time of the deodorizing fan 330 with a reference time (665).

When the cumulative drive time of the deodorizing fan 330 is the reference time or more, the refrigerator 200 displays replacement of the deodorizing filter 100 on the display unit 520 (670).

When the cumulative drive time of the deodorizing fan 330 is less than the reference time or more, the refrigerator 200 repeats calculation of the cumulative drive time of the deodorizing fan 330 and comparison between the calculated cumulative drive time and the reference time until the cumulative drive time of the deodorizing fan 330 becomes the reference time or more.

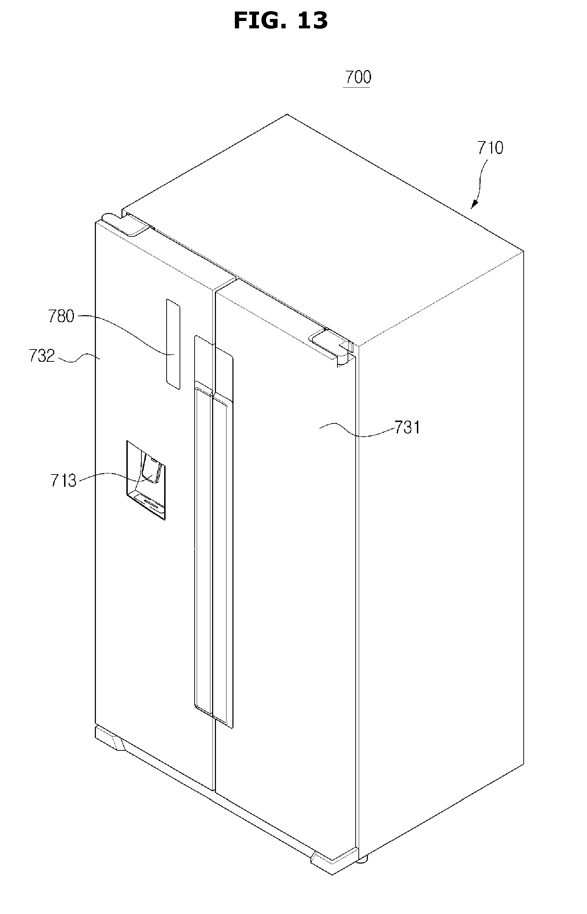

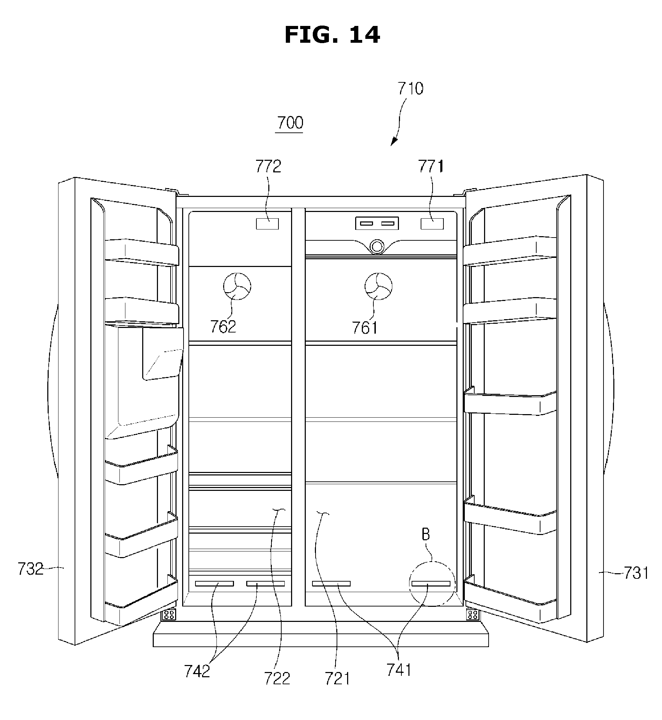

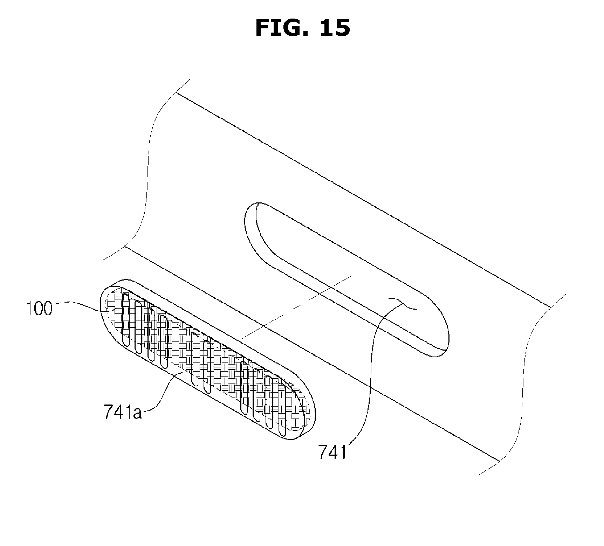

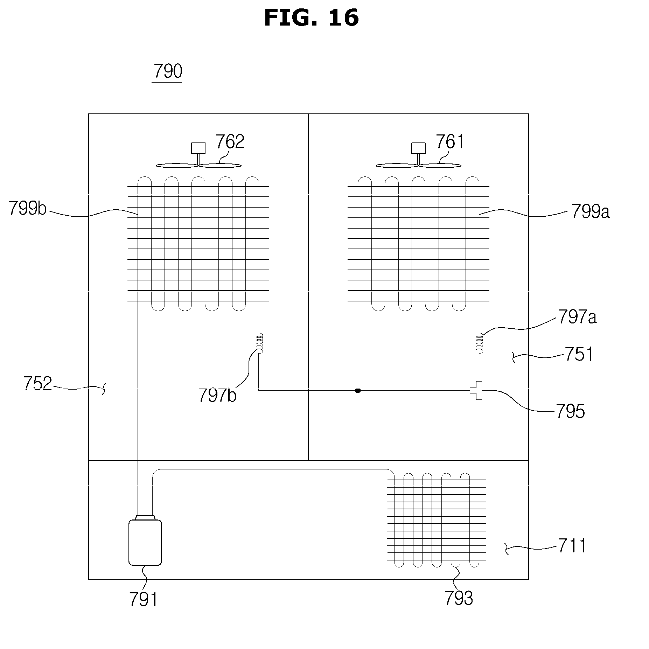

FIG. 13 is view showing an external appearance of another exemplary refrigerator having the deodorizing filter in accordance with the embodiment of the disclosure, FIG. 14 is a front view showing the another exemplary refrigerator having the deodorizing filter in accordance with the embodiment of the disclosure, FIG. 15 is an enlarged view of portion B of FIG. 14, and FIG. 16 is a view showing a cooling device of the another exemplary refrigerator having the deodorizing filter in accordance with the embodiment of the disclosure.

Referring to FIGS. 13 to 16, the refrigerator 700 includes a main body 710 defining an external appearance of the refrigerator 700, storage compartments 721, 722 defined in the main body 710 for storage of food, doors 731, 732 to shield the storage compartments 721, 722 from the outside, and a cooling device 790 to cool the storage compartments 721, 722.

The storage compartments 721, 722 include the refrigerating compartment 721 and the freezing compartment 722. A first temperature sensor 771 is installed in the refrigerating compartment 721 to detect a temperature of the refrigerating compartment 721, and a second temperature sensor 772 is installed in the freezing compartment 722 to detect a temperature of the freezing compartment 722.

The refrigerating compartment 721 is provided at a rear face thereof with a first cooling duct 751, through which air of the refrigerating compartment 721 flows, and a first evaporator 799a that will be described hereinafter is installed in the first cooling duct 751. The refrigerating compartment 721 has a first suction port 741 formed near the bottom thereof, through which air of the refrigerating compartment 721 is suctioned into the first cooling duct 751, and a first cooling fan 761 installed in an upper region of the refrigerating compartment 721, by which air of the first cooling duct 751 is discharged into the refrigerating compartment 721.

A deodorizing filter case 741a, in which the deodorizing filter 100 to adsorb odor particles contained in air is received, is fitted into the first suction port 741. The deodorizing filter 100, fitted into the first suction port 741, adsorbs odor particles contained in air suctioned through the first suction port 741. A configuration and a manufacturing method of the deodorizing filter 100 have been described above with reference to FIGS. 1A to 1C and FIGS. 2A to 2D, and thus a description thereof will be omitted hereinafter.

The deodorizing filter case 741a has the same shape and size as those of the first suction port 741, so as to be coupled to or separated from the first suction port 741. The deodorizing filter case 741a has pass-through pores to allow air of the refrigerating compartment 721 to smoothly pass through the first suction port 741. The deodorizing filter case 741a may be formed of a synthetic resin or a metal.

The deodorizing filter 100 is received in the deodorizing filter case 741a. Thus, the air having passed through the pass-through pores of the deodorizing filter case 741a is deodorized by the deodorizing filter 100 prior to being introduced into the first cooling duct 751.

The freezing compartment 722 includes a second cooling duct 752, a second suction port 742, and a second cooling fan 762, configurations and functions of which are equal to those of the first cooling duct 751, the first suction port 741, and the first cooling fan 761.

The doors 731, 732 are installed to the front face of the main body 710 to shield the storage compartments 721, 722 from the outside. The doors 731, 732 are provided at front faces thereof with a dispenser 713 to discharge purified water and a control panel 780 to receive an operating instruction for the refrigerator 700 from the user and display operating information of the refrigerator 700. The control panel 780 will be described below in detail.

The cooling device 790 includes a compressor 791, a condenser 793, a flow path switching valve 795, expansion valves 797a, 797b, and evaporators 799a, 799b. The compressor 791, the condenser 793, the flow path switching valve 795, the expansion valves 797a, 797b, and the evaporators 799a, 799b have the same configurations and functions as those of the compressor 291, the condenser 293, the flow-path switching valve 295, the expansion valves 297a, 297b, and the evaporators 299a, 299b described above with reference to FIG. 5, and thus a description thereof will be omitted hereinafter.

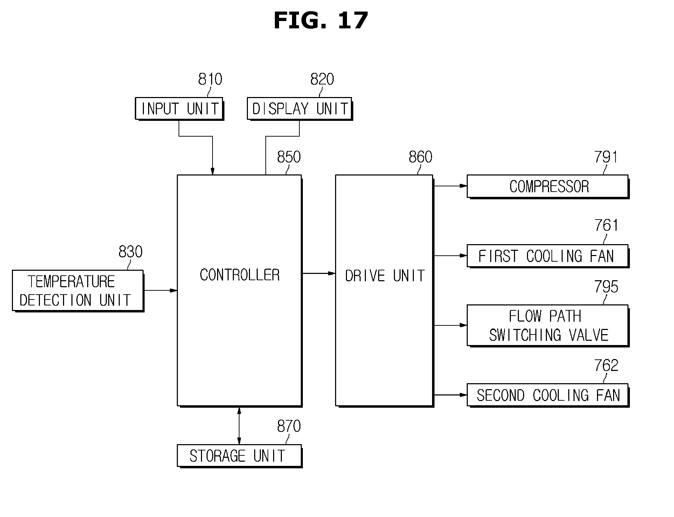

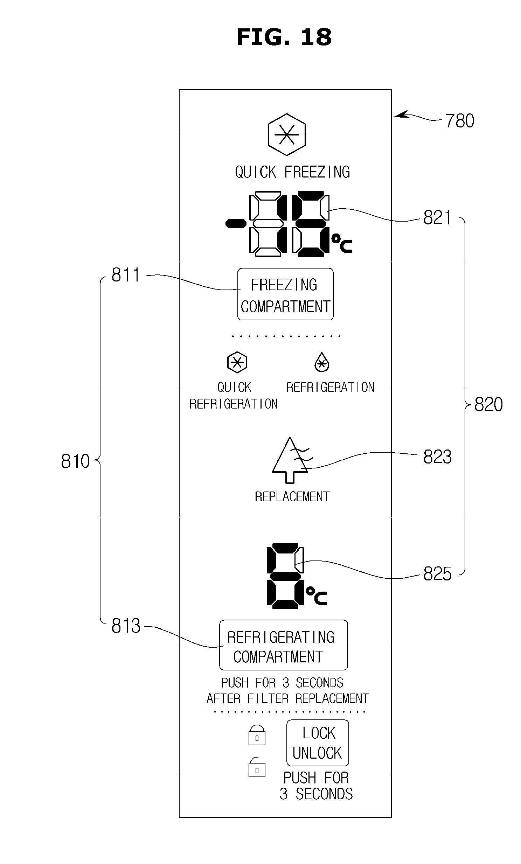

FIG. 17 is a block diagram showing control flow of the another exemplary refrigerator having the deodorizing filter in accordance with the embodiment of the disclosure, and FIG. 18 is a detailed view showing a control panel shown in FIG. 13.

Referring to FIGS. 17 and 18, to perform a function thereof, the refrigerator 700 includes an input unit 810, a display unit 820, a temperature detection unit 830, a controller 850, a drive unit 860, a storage unit 870, the compressor 791, the first cooling fan 761, the second cooling fan 762, and the flow path switching valve 795.

The compressor 791, the first cooling fan 761, and the second cooling fan 762 described above will not be repeatedly described hereinafter.

The display unit 820 and the input unit 810 that will be described hereinafter constitute the control panel 780, and the display unit 820 displays operating information of the refrigerator 700. The display unit 820 includes a freezing-compartment temperature display area 821 to display a temperature of the freezing compartment 722, a refrigerating-compartment temperature display area 825 to display a temperature of the refrigerating compartment 721, and a deodorizing-filter replacement display area 823 to display a replacement point in time of the deodorizing filter 100.

Along with the display unit 820 described above, the input unit 810 constitutes the control panel 780, and receives an operating instruction for the refrigerator 200 from the user. In addition, the input unit 810 includes a freezing-compartment operating-instruction input button 811 to set a target temperature of the freezing compartment 722 and an operating mode of the freezing compartment 722, and a refrigerating-compartment operating-instruction input button 813 to set a target temperature of the refrigerating compartment 721 and an operating mode of the refrigerating compartment 721.

When the deodorizing-filter replacement display area 823 displays an instruction to replace the deodorizing filter 100, the user may initialize a deodorizing-filter replacement point in time via the refrigerating-compartment operating-instruction input button 813. For example, when the deodorizing-filter replacement display area 823 of the display unit 820 displays "Replacement" of the deodorizing filter 100, the user may initialize a replacement point in time of the deodorizing filter 100 by pushing the refrigerating-compartment operating-instruction input button 813 for several seconds after replacing or reproducing the deodorizing filter 100 of the deodorizing module 300.

The temperature detection unit 830 includes the first temperature sensor 771 and the second temperature sensor 772 installed respectively in the refrigerating compartment 721 and the freezing compartment 722. The temperature detection unit 830 detects temperatures of the storage compartments 721, 722 and outputs signals corresponding to the detected temperatures to the controller 850.

The drive unit 860 generates drive current to drive the compressor 791, the first cooling fan 761 and the second cooling fan 762 and drive current to open or close the flow path switching valve 795 based on a control signal of the controller 850 that will be described hereinafter.

The storage unit 870 may store programs and data to control the refrigerator 700 and may also temporarily store operating information of the refrigerator 700.

The controller 850 controls overall operation of the refrigerator 700. In particular, the controller 850 compares a cumulative drive time of the first cooling fan 761 with a reference time. When the cumulative drive time of the first cooling fan 761 is a reference time or more, the controller 850 controls the display unit 820 to display replacement of the deodorizing filter 100.

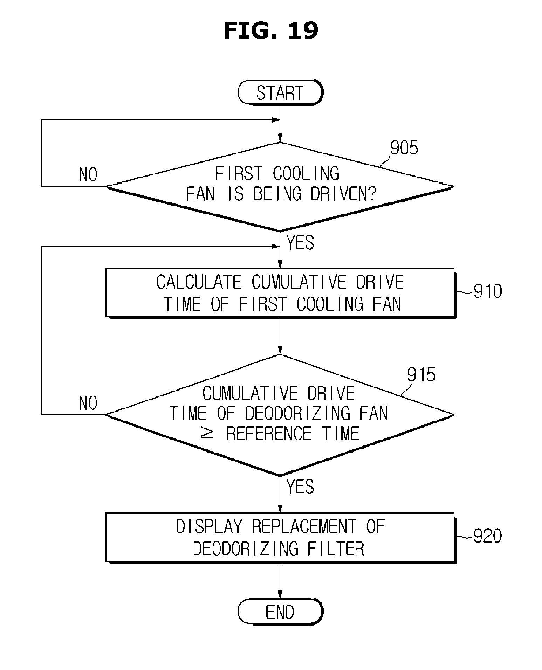

FIG. 19 is a flowchart showing a method of displaying replacement of the deodorizing filter by the another exemplary refrigerator having the deodorizing filter in accordance with the embodiment of the disclosure.

Referring to FIG. 19, the refrigerator 700 judges whether or not the first cooling fan 761 is being driven (905).

When the first cooling fan 761 is driven, the refrigerator 700 calculates a cumulative drive time of the first cooling fan 761 (910).

Next, the refrigerator 700 compares the cumulative drive time of the first cooling fan 761 with a reference time (915).

When the cumulative drive time of the first cooling fan 761 is the reference time or more, the refrigerator 700 displays replacement of the deodorizing filter 100 on the display unit 820 (920).

When the cumulative drive time of the first cooling fan 761 is less than the reference time or more, the refrigerator 700 repeats calculation of the cumulative drive time of the first cooling fan 761 and comparison between the calculated cumulative drive time and the reference time until the cumulative drive time of the first cooling fan 761 becomes the reference time or more.

As is apparent from the above description, according to one aspect of the present disclosure, more effective deodorization for interior air of a refrigerator may be accomplished owing to an increased contact area between deodorizer materials of a deodorizing filter and the interior air of the refrigerator.

Although the embodiments of the present disclosure have been shown and described, it would be appreciated by those skilled in the art that changes may be made in these embodiments without departing from the principles of the invention, the scope of which is defined in the claims.

## Claims

1. A refrigerator comprising at least one deodorizing filter to adsorb particles contained in fluid,
wherein the deodorizing filter comprises:
a substrate having plural pass-through pores to allow passage of the fluid;
an adherent material applied to a surface of the substrate; and
a plurality of porous deodorizer materials fixed to the surface of the substrate by the adherent material to adsorb the particles.

2. The refrigerator according to claim 1, wherein spaces to allow passage of the fluid are defined between the porous deodorizer materials.

3. The refrigerator according to claim 1 or 2, wherein the substrate is a wire mesh.

4. The refrigerator according to claim 3, wherein the adherent material is applied to wires of the mesh so that the porous deodorizer materials fix on the wires of the mesh.

5. The refrigerator according to claim 3 or 4, wherein each of the deodorizer materials includes at least one of activated charcoal, carbon fiber, zeolite, and silica.

6. The refrigerator according to claim 3 or 4, wherein each of the deodorizer materials is formed by applying a metal oxide or metal sulfate to at least one from among activated charcoal, carbon fiber, zeolite, and silica.

7. The refrigerator according to claim 6, wherein the metal oxide or the metal sulfate includes at least one from among manganese oxide (MnO), copper oxide (CuO), and copper sulfate (CuSO₄).

8. The refrigerator according to claim 3 or 4, wherein each of the deodorizer materials includes activated charcoal formed by applying an organic acid or an inorganic acid to at least one from among activated charcoal, carbon fiber, zeolite, and silica.

9. The refrigerator according to claim 8, wherein the organic acid or the inorganic acid includes at least one from among sulfuric acid (H₂SO₄) and phosphoric acid (H₃PO₄).

10. The refrigerator according to any one of the preceding claims, further comprising:
at least one storage compartment;
a cooling device to cool the storage compartment; and
a deodorizing fan installed in the flow path to move the air such that the air passes through the deodorizing filter.

11. The refrigerator according to claim 10, wherein the deodorizing fan is located downstream of the deodorizing filter, and moves the air having passed through the deodorizing filter into the storage compartment.

12. The refrigerator according to claim 10 or 11, further comprising a particle concentration detection unit to detect a concentration of particles contained in the interior air of the storage compartment.

13. The refrigerator according to claim 12, further comprising a controller to change a drive rate of the deodorizing fan based on a detection result of the particle concentration detection unit.

14. The refrigerator according to claim 13, wherein the controller increases the drive rate of the deodorizing fan when the concentration of particles is a first reference concentration or more.

15. The refrigerator according to claim 13 or 14, wherein the controller reduces the drive rate of the deodorizing fan when the concentration of particles is a second reference concentration or less.
